Europäisches Patentamt

European Patent Office

Office européen des brevets

(19)

(11) Veröffentlichungsnummer: **0 355 397**
**A1**

(12) # EUROPÄISCHE PATENTANMELDUNG

(21) Anmeldenummer: **89113227.6**

(22) Anmeldetag: **19.07.89**

(51) Int. Cl.4 **A61F 5/56 , A61G 7/00 , A47C 19/00**

(30) Priorität: **17.08.88 DE 3827878**
**17.09.88 DE 3831699**
**25.10.88 DE 3836292**

(43) Veröffentlichungstag der Anmeldung:
**28.02.90 Patentblatt 90/09**

(84) Benannte Vertragsstaaten:
**AT BE CH DE ES FR GB GR IT LI LU NL SE**

(71) Anmelder: **Fuchs, Sieglinda**
**Auf der Hofreith 20**
**D-4000 Düsseldorf 31(DE)**

(72) Erfinder: **Fuchs, Sieglinda**
**Auf der Hofreith 20**
**D-4000 Düsseldorf 31(DE)**

(74) Vertreter: **Langmaack, Jürgen, Dipl.-Ing. et al**
**Patentanwälte Maxton . Maxton . Langmaack**
**Goltsteinstrasse 93,VII Postfach 51 08 06**
**D-5000 Köln 51(DE)**

(54) **Bett für Schnarcher.**

(57) Das Problem, bei Schläfern das Schnarchen zu verhindern, sollte bisher durch Geräte bewirkt werden, die unmittelbar am Körper angebracht sind und diesen in eine Seitenlage zwingen. Hierdurch ist ein entspanntes Schlafen nicht möglich.

Durch eine ebenflächige, um eine Längsachse mittels steuerbarem Antrieb aus der Horizontalen in eine Schräglage verschwenkbare Matratzenunterstützung (8) wird der schnarchende Schläfer ohne aufgeweckt zu werden selbsttätig in die Seitenlage gerollt, so daß das Schnarchen aufhört.

Da das Bett nach kurzer Zeit wieder in die Horizontale verschwenkt werden kann, ist auch für den Schnarcher ein ungestörtes, entspanntes Schlafen gewährleistet.

Fig. 1

EP 0 355 397 A1

## Bett für Schnarcher

Die Erfindung betrifft ein Bett zum Beeinflussen des Schnarchens, das eine um wenigstens eine Längsachse verschwenkbar ausgebildete Matratzenunterstützung mit im wesentlichen ebenflächiger Liegefläche aufweist, die mit einem vorzugsweise elektromotorischen Schwenkantrieb verbunden ist, der über eine Steuerschaltung an eine Stormversorgung angeschlossen ist.

Es ist in der Vergangenheit in vielfältiger Weiser versucht worden, das Schnarchen schlafender Menschen zu verhindern, da die durch das Schnarchen erzeugten Geräusche jede, mit dem Schlafenden gemeinsam im selben Raum schlafende Person erheblich stört, wobei mitunter die Schnarchgeräusche eine solche Lautstärke erreichen können, daß sogar der Schlafende selbst aufwacht. Wenn man den Schnarcher weckt, hört das Schnarchen zwar auf, er ist jedoch in seinem Schlaf gestört. Nach dem Einschlafen beginnt in den meisten Fällen jedoch kurz darauf die Schnarcherei von neuem. Es hat sich nun in der Mehrzahl der Fälle erwiesen, daß die Schnarchgeräusche nicht entstehen, wenn und solange der Betreffende in der Seitenlage ruht. Um eine Seitenlage zu erzwingen, ist aus der DE-AS 11 98 005 ein Gerät bekannt, das aus einem gepolsterten Brett von der Länge des Oberkörpers besteht, das in der Längsachse klappbar ausgebildet und mit einer Feststelleinrichtung versehen ist, die den hochklappbaren Teil unter einem Winkel zwischen 60° und 90° zu fixieren vermag. Das Gerät ist an seinen beiden Stirnenden mit Schlaufen versehen, durch die ein Bein und ein Arm hindurchgesteckt werden, so daß der Schlafende bei hochgeklapptem Teil zwangsweise in der Seitenlage liegt. Abgesehen von der nicht nachgiebigen Körperunterstützung ist ein entspanntes Schlafen mit einem derartigen Gerät nicht möglich, da der Schlafende auf eine Seitenlage fixiert ist und sich nicht umzudrehen vermag. Durch die Armschlaufe ist noch nicht einmal ein freier Bewegungsablauf in der Seitenlage, beispielsweise eine Unterstützung des Kopfes durch einen Arm möglich. Ein weiterer Nachteil des vorbekannten Gerätes besteht darin, daß durch die um den Oberschenkel und um die Schulter angebrachte Schlaufe die Blutzirkulation beeinträchtigt werden kann.

Aus der US-PS 3 089 130 ist eine Einrichtung an einem Bett bekannt, bei der der Kopf des Schläfers auf einer schwenkbaren und mit einem Stoßantrieb versehenen Kopfstütze gelagert ist. Schnarchgeräusche werden über ein Mikrofon aufgenommen und über ein Steuersignal einer Steuereinrichtung zugeleitet, die dann den Stoßantrieb aktiviert. Durch den Stoßantrieb wird der Kopf des Schläfers auf- und abgeschüttelt, so daß dieser aufwacht und das Schnarchen einstellt. Eine derartige Einrichtung beendet zwar das Schnarchen sehr wirksam, ist jedoch in hohem Maße ungesund, da der Schlaf des Schnarchers immer wieder unterbrochen wird.

Aus der DE-AS 11 51 347 ist eine ähnliche Einrichtung vorbekannt, bei der jedoch der auf einer horizontal bewegbaren Unterlage ruhende Kopf des Schläfers über eine Antriebsein richtung hin- und herbewegt wird, wobei die Einschaltung der Antriebseinrichtung wiederum über ein Mikrofon ausgelöst wird. Bei dieser Einrichtung wird über ein hin- und herbewegbares horizontales Förderband bzw. eine horizontal hin- und herschiebbare Platte ausschließlich der Kopf ohne Rücksicht auf die übrige Körperhaltung zur Seite gebogen, so daß infolge der nicht begrenzten Kraftwirkung Schädigungen des Halswirbels zu befürchten sind. Die gerade für die Schlafhaltung unnatürliche, durch die Vorrichtung erzwungene Seitenneigung des Kopfes muß zwangsläufig zu einer Schlafunterbrechung führen, so daß auch hier neben der Gefahr von Beschädigungen des Halswirbelsystems ebenfalls ein ungestörter Schlaf nicht gewährleistet ist.

Aus der WO 86/03663 ist eine in Längsrichtung dreigeteilte Matratze bekannt, deren Mittelteil auf einem Wippbrett federnd abgestützt ist. Die Rükkenlage kann hierbei nur dann eingenommen werden, wenn der Schläfer auf dem Mittelstreifen genau über der Kipprolle liegt. Bei jeder Verlagerung des Körpers mit geringem seitlichem Abstand zur Kippunterstützung kippt das Wippbrett zur Seite ab, so daß das Mittelteil und das anschließende, ebenfalls federnd abgestützte Seitenteil eine im Querschnitt V-förmige Ausrichtung einnehmen. Liegt nun der Schläfer in der Rückenlage etwa auf der Nahtstelle zwischen Mittelteil und Seitenteil der Matratze, dann sind diese beiden aneinandergrenzenden Teile in etwa V-förmig eingeknickt, so daß die Matratze insgesamt die Wirkung der berüchtigten "Kuhle" einer alten durchgelegenen Matratze aufweist. Liegt ein Schnarcher erst einmal in Rükkenlage auf dem V-förmig abgeknickten Grenzbereich zwischen Mittel-und Seitenteil, dann hat er keinerlei Möglichkeit mehr, sich in die das Schnarchen beendenden Seitenlage zu drhen oder durch einen Partner drehen zu lassen, da wegen der Wirkung des Wippbrettes der Körper immer "bergauf" bewegt werden muß. Der gleiche Nachteil haftet der aus der DE-PS 560 715 bekannt gewordenen Bettkonstruktion an, die eine V-förmig einknickbare Matratzenunterstützung aufweist.

Aus US-PS 3 013 281, DE-GM 18 37 239, DE-OS 26 36 746 sowie DE-OS 24 08 784 sind Krankenhausbetten bekannt, die eine im wesentlichen trogförmige Liegefläche aufweisen und bei denen die Matratze bzw. die Matratzenunterstützung um die Längsachse verschwenkbar ist. Derartige Betten dienen in erster Linie zur Unterstützung des Pflegepersonals beim Umbetten, sollen aber auch die Belastung einzelner Körperpartien variieren, wobei durch den Schwenkvorgang der Körper des Patienten aus medizinischen Gründen, beispielsweise bei Frakturen oder dgl. seine Lage relativ zur Liegefläche nicht verändern darf. In gegebenen Fällen soll bei bettlägerigen Personen durch Schaukelbewegungen der Liegefläche eine Unterstützung des Kreislaufs bewirkt werden (DE-GM 18 37 239 und DE-OS 24 08 784). Keines dieser vorbekannten Betten ist für die Verwendung im häuslichen Bereich geeignet, da es sich praktisch um sogenannte Trogbetten handelt, die die liegende Person in einer vorgegebenen Lage fixieren sollen, während es gerade ein wesentlicher Gesichtspunkt eines Bettes für normale Wohnzwecke ist, daß hier bei der Benutzung eine uneingeschränkte Bewegungsmöglichkeit durch eine praktisch ebenfläche Liegefläche gegeben ist, die auch für den Schläfer vorhanden sein muß, damit dieser auch während des Schlafs ungehindert eine andere Liegehaltung einnehmen kann, beispielsweise von einer Seitenlage über die Rückenlage in die andere Seitenlage sich umwenden kann.

Zusammenfassend ergibt sich, daß die speziell zur Verhinderung des Schnarchens bisher entwikkelten Einrichtungen für den täglichen Gebrauch ungeeignet sind. Soweit Betten bekannt sind, die zumindest mit Teilen der Matratze bzw. der Matratzenunertstützung um eine Längsachse schwenkbar ausgebildet sind, sind diese für die Bedürfnisse des Krankenhauses oder der Pflege bettlägeriger Personen konzipiert und so ausgebildet, daß die Verwendung als normales Bett für häusliche Bedürfnisse nicht gegeben ist und darüber hinaus die ungehinderte Bewegung eines Schläfers in erheblichem Maße beeinträchtigt wird.

Der Erfindung liegt die Aufgabe zugrunde, ein Bett der eingangs bezeichneten Art zu schaffen, das ohne oder nur bei geringster Behinderung der Bewegungsfreiheit des Schlafenden ein Schnarchen verhindert bzw. kurzfristig nach dem Einsetzen des Schnarchens beendet. Die Form muß hierbei so gestaltet sein, daß die Einrichtung im Wohnbereich eingesetzt werden kann und allen Wohnbedürfnissen gerecht wird.

Diese Aufgabe wird erfindungsgemäß dadurch gelöst, daß der Matratzenunterstützung vorzugsweise berührungslos arbeitende Endlagenschalter zugeordnet sind, die jeweils bei Erreichen der Schräglage oder der Horizontallage den Antrieb abschaltet. Ein derart ausgebildetes Bett hat den Vorteil, daß der Schläfer das Bett ohne jede Behinderung seiner Bewegungsfreiheit benutzen und nach Belieben die Rückenlage und jede Seitenlage einnehmen kann. In der überwiegenden Zahl der Fälle beginnen Menschen beim Einsetzen des Tiefschlafes zu schnarchen und zwar dann, wenn sie sich in der Rückenlage befinden. Eine im gleichen Raum schlafende und durch das Schnarchgeräusch gestörte Person kann nun über einen entsprechenden Schalter, vorzugsweise einem einfachen Tastschalter, den Getriebemotor starten, was auch im Halbschlaf geschehen kann, so daß nunmehr die Matratzenunterstützung mitsamt Matratze und Schläfer in eine vorgegebene Schrägstellung hochgeschwenkt wird. Es hat sich nun überraschend gezeigt, daß bei einer Schräglage zwischen 10° und 25° ein in der Rückenlage liegender Schläfer sich innerhalb kurzer Zeit von selbst in die Seitenlage rollt ohne hierdurch aufzuwachen. Hält man das Bett über einen Zeitraum von einigen Minuten in dieser Schräglage, dann nimmt der Schläfer von sich aus eine stabile Seitenlage ein, so daß dann das Bett von Hand wieder in die Horizontallage zurückgeschwenkt werden kann. Besonders zweckmäßig ist es jedoch, wenn die Steuerschaltung mit einer Zeitschaltuhr gekoppelt ist, die nach Ablauf einer vorgebbaren Zeit den Schwenkantrieb auf Rücklauf in die Horizontale einschaltet. Damit nimmt das Bett wieder seine normale Lage ein ohne daß der Schläfer sofort wieder sich in die Rücken lage dreht. Andererseits ist der darauf befindliche Schläfer nicht daran gehindert, sich frei zu bewegen und nach einiger Zeit eine andere Schlaflage einzunehmen. Bei einem Schlaf in Seitenlage wird vielfach nach einiger Zeit die andere Seitenlage gewählt, so daß trotz der horizontalen Lage der Liegefläche der betreffende Schläfer nicht sofort wieder mit dem Schnarchen beginnt. Andererseits braucht die die Schwenkbewegung auslösende Person nicht darauf zu achten, daß das Bett nach dem Einstellen des Schnarchens wieder in die Normallage zurückgeschwenkt wird. Dieses erfolgt vielmehr automatisch. Die Verwendung eines Elektromotors, vorzugsweise eines elektrischen Getriebemotors, hat den Vorteil, daß hier mit kleinen Motoren mit geringer elektrischer Leistung praktisch ohne störendes Laufgeräusch der Schwenkvorgang ausgeführt werden kann. Die Schwenkachse der Matratzenunterstützung kann hierbei in der einfachsten Form an einer Längsseite angeordnet sein, so daß die Matratzenunterstützung mit der anderen Längsseitenkante je nach der zur Verfügung stehenden Höhe über dem Fußboden aus der Horizontallager durch Verschwenken nach unten oder Verschwenlen nach oben in die Schräglage verbracht werden kann.

Bei einem Bett mit einer um wenigstens eine Längsachse, vorzugsweise um die Mittellängsachse aus der Horizontallage in beide Richtungen verschwenkbar gelagerten Matratzenunterstützung ist in vorteilhafter Ausgestaltung der Erfindung ferner ein lastfühlender Umschalter vorgesehen, der jeweils die Richtung einer von der Horizontallage ausgehenden Schwenkbewegung gegen das von einer einseitigen Auflast auf der Matratzenunterstützung vorgegebenen Lastmoment steuert. Da die Rückenlage von einem Schläfer nicht immer genau im Bereich der Mittenlängsachse der Matratzenunterstützung bzw. der Matratze eingenommen wird, sondern durchaus auch nur auf einer der beiden Seitenhälften der Matratze eingenommen werden kann, bietet diese Ausgestaltung den Vorteil, daß für die Schräglage die Matratzenunterstützung auf der Seite hochgeschwenkt wird, auf der der betreffende Schläfer liegt, so daß ein ausreichender Bewegungsraum zum Einnehmen der Seitenlage vorhanden ist. Der lastfühlende Umschalter ist je nach der Konstruktion des Schwenkantriebes im Bereich zwischen Matratzenunterstützung und der Abstützung des Schwenkantriebes am Bettkasten anzuordnen, so beispielsweise im Bereich der Befestigung des Motors am Bettkasten, da an dieser Stelle das Lastmoment als Zug- oder Druckkraft erfaßbar ist. Die Umschaltung kann auch durch die Erfassung der Lastaufnahme des Motors bewirkt werden. Die Matratzenunterstützung kann hierbei entweder an beiden Längsrändern gelagert sein, so daß sie jeweils nach der einen oder anderen Seite hochschwenkbar ist, oder aber um die Mittellängsachse nach beiden Seiten verschwenkbar gelagert sein.

Die Matratzenunterstützung kann stirnseitig mit je einem Wellenzapfen am Bettkasten oder einem gesonderten Stützgestell gelagert sein. Hierbei kann der Antriebsmotor auch unmittelbar das Drehmoment über einem Wellenzapfen einleiten, wobei ein entsprechendes Getriebe vorgesehen sein muß. Eine Schwenkbarkeit um eine Längsachse kann auch dadurch bewirkt werden, daß die Matratzenunterstützung stirnseitig über jeweils zwei Schwenkhebel mit dem Bettkasten oder dessen Stützgestell verbunden ist, wobei die beiden Schwenkhebel nicht parallel verlaufen dürfen, sondern in der Schwenkebene unter einem Winkel zueinander ausgerichtet sein müssen. Die Matratzenunterstützung kann hierbei mit den Schwenkhebeln "hängend", aber auch auf diesen "stehend" verbunden sein. Diese Bauform bildet jeweils an der Stirnseite eine sogenannte Vier-Gelenk-Kette, so daß durch die Bemessung der Winkelstellung der Hebel zueinander, deren Länge und der Gelenkabstände an der Matratzenunterstützung bzw. am Bettkasten Einfluß auf den Schwenkwinkel und seine Abhängigkeit vom Antriebshub, beispielsweise einer Schubstange genommen werden kann. Anstatt einer einteiligen starren Matratzenunterstützung kann diese auch längsgeteilt sein, so daß entsprechend der Ansteuerung des Motors durch den lastabhängigen Umschalter die eine Längshälfte oder die andere Längshälfte hochgeschwenkt und nach entsprechendem Zeitablauf wieder zurückgeschwenkt wird.

In zweckmäßiger Ausgestaltung der Erfindung ist als Schwenkantrieb ein elektrischer Getriebemotor vorgesehen, der auf eine Schubstange einwirkt. Mit einem derartigen Schwenkantrieb lassen sich auch mit kleineren Elektromotoren die erforderlichen Drehmomente für den Schwenkvorgang erzeugen, wobei der Schwenkvorgang in verhältnismäßig kurzer Zeit beendet ist, so daß der Schläfer die gewünschte Seitenlage alsbald einnimmt. Die Schubstange kann als Zahnstange oder als Schraubspindel ausgebildet sein. Ein Antrieb über eine Schraubspindel hat den Vorteil einer größeren Laufruhe und eines größeren Übersetzungsnisses, so daß auch mit kleinen Motoren große Drehmomente über die Schubstange ausgeübt werden können.

In zweckmäßiger Ausgestaltung der Erfindung ist ferner vorgesehen daß der Getriebemotor stirnseitig unter der Matratzenunterstützung und mit Abstand zur Schwenkachse angeordnet ist. Diese Anordnung hat den Vorteil, daß die normale Baulänge eines Bettes nicht überschritten wird, da für die Anordnung des Getriebemotors der ohnehin vorhandene Freiraum unterhalb der Matratzenunterstützung ausgenutzt wird. Durch eine entsprechende Wahl des Abstandes des Getriebemotors von der Schwenkachse kann hier das aufzubringende Drehmoment erheblich reduziert werden. Der Motor sollte in möglichst großem Abstand von der Schwenkachse angebracht werden.

In einer bevorzugten Ausgestaltung der Erfindung ist hierbei vorgesehen, daß insbesondere bei einer um die Mittenlängsachse schwenkbar gelagerten Matratzenunterstützung die Schubstange mit dem die Richtung des Lastmomentes vorgegeben lastfühlenden Umschalter der Steuerschaltung verbunden ist. Bei dieser Ausgestaltung wird mit Vorteil ausgenutzt, daß in bezug auf die Querrichtung die mittig gelagerte Matratzenunterstützung nach Art eines Waagebalkens wirkt, so daß der Schwenkantrieb "fühlt", auf welcher Matratzenhälfte der Schläfer liegt. Setzt nun das Schnarchen ein, so wird der Schwenkantrieb in seiner Drehrichtung so eingeschaltet, daß die belastete Matratzenhälfte nach oben verschwenkt wird, so daß wiederum gewährleistet ist, daß sich der Schläfer in Richtung auf die freie Betthälfte in die Seitenlage dreht. Dieser Effekt läßt sich auch nutzen, wenn die Matratzenunterstützung jeweils an den Längsrändern mit den Schwenklagern versehen ist und jede Längsseite mit einem Hubantrieb verbunden ist. Zum Schwenken wird dann der Antrieb geschaltet, auf dem das größte Lastmoment wirkt.

In einer besonders vorteilhaften Ausgestaltung der Erfindung ist ein elektro-akustischer Sensor vorgesehen, der auf die Steuerschaltung aufgeschaltet ist und der bei Schnarchlauten den Schwenkantrieb einschaltet. In ruhigen Wohnlagen kann dieser elektro-akustische Sensor durch ein einfaches Mikrofon gebildet werden, dem eine entsprechender Steuerschaltung zugeordnet ist, die beim Überschreiten eines bestimmten Schallpegels den Schwenkantrieb einschaltet. Die elektro-akustische Sensorschaltung kann aber auch so ausgebildet werden, daß der Schwenkantrieb erst dann eingeschaltet wird, wenn über einen gewissen Zeitraum Geräusche mit einer vorgegebenen Lautstärke und innerhalb eines bestimmten Frequenzbandes, nämlich dem für das Schnarchen übliche niedrige Frequenzband erfaßt werden. Diese Anordnung hat den Vorteil, daß der Schwenkantrieb über die Schnarchgeräusche eingeschaltet wird. Hierdurch wird nicht nur eine im gleichen Raum schlafende Person der Notwendigkeit enthoben, einen Schalter zu betätigen. Der Vorteil liegt insbesondere darin, daß auch bei einem alleinschlafenden Schnarcher das Schnarchen kurzfristig beendet wird. Dies ist deshalb wichtig, weil nach neueren medizinischen Untersuchungen das Schnarchen auch für den Schnarcher selbst mit gesundheitlichen Nachteilen verbunden ist.

Eine bevorzugte Ausgestaltung sieht vor, daß zu beiden Längsseiten der Matratzenunterstützung feststehende Halteelemente für den Rand der Matratze angeordnet sind. Diese Anordnung hat den Vorteil, daß ohne zusätzliche bewegliche Teile jeweils auf der nach unten schwenkenden Seite die Matratze im Randbereich über die Halteelemente in ihrer ursprünglichen Lage gehalten wird und so eine in etwa horizontale Restfläche zur Unterstützung des Körpers in der Seitenlage bestehen bleibt. Die Flexibilität einer modernen Matratze reicht aus, um einen sanften Übergang von der schrägen Fläche in die im wesentlichen horizontale Randfläche zu bilden. Die Halteelemente sind hierbei kammartig an feststehenden Teilen des Bettes entlang beider Längsseiten befestigt.

Insbesondere ist in Ausgestaltung der Erfindung vorgesehen, daß die Matratzenunterstützung in an sich bekannter Weise als Lattenrost mit quer zur Längsachse im Abstand zueinander angeordneten Latten ausgebildet ist und daß am Bett den Längsrändern der Matratzenunterstützung zugeordnete Halteelemente angeordnet sind, die jeweils in den Zwischenraum zwischen zwei Latten von unten hineinragen und bei Horizontalstellung der Matratzenunterstützung mit ihren oberen Enden im wesentlichen in der Ebene des Lattenrostes liegen. Diese Ausgestaltung läßt sich auch in der Weise abwandeln, daß der Lattenrost selbst in seiner ursprünglichen Lage verbleibt und lediglich durch entsprechend ausgebildete und mit einem Antrieb verbundene Halteelemente die Matratze vom Rand her angehoben und über eine ausreichende Breite in die erforderliche Schräglage verbracht wird, so daß der Körper des Schlafenden in die Seitenlage rollen kann. Auch hier sind den jeweiligen Antrieben lastfühlende Sensoren zugeordnet, so daß die Matratze beim Auftreten von Schnarchgeräuschen an der "richtigen" Seite angehoben wird. Diese Anordnung kann auch so getroffen werden, daß sich die Halteelemente über ihren Antrieb unmittelbar auf dem Boden des Raumes bzw. des Bettkastens abstützen, so daß durch entsprechende Höhenänderung das Hochschwenken einer Matratzenseite bewirkt wird. In Abwandlung dieser Konzeption ist es auch im Rahmen der Erfindung möglich, die Matratzenunterstützung selbst mit wenigstens zwei in ihrer Höhe mittels eines Antriebs veränderbaren Füßen abzustützen, durch die dann der Schwenkvorgang bewirkt werden kann. Bei der Anordnung von vier höhenverän derbaren Füßen, von denen jeweils die an einer Längsseite angeordneten Füße synchron antreibbar sind, kann je nach Konstruktion auf eine besondere Schwenklagerung für die Matratzenunterstützung verzichtet werden.

In Ausgestaltung der Erfindung ist ferner vorgesehen, daß die Matratzenunterstützung am Rand der beim Schwenken die Horizontalebene überragenden Bereiche mit einer nach unten weisenden, in den Bettkasten reichenden Abdeckschürze versehen ist. Eine derartige, zumindest an den Längsseiten angebrachte Abdeckschürze gewährleistet, daß in hochgeschwenktem Zustand nicht in den sich öffnenden Spalt zwischen Matratzenunterstützung und dem Rand des Bettkastens hineingegriffen werden kann, so daß Verletzungen beim Abschwenken ausgeschlossen sind.

Gemäß der Erfindung wird für ein Bett der eingangs bezeichneten Art ein Nachrüstsatz vorgeschlagen, der gekennzeichnet ist durch zwei Stirnteile, die jeweils ein Schwenklager für die Matratzenunterstützung aufweisen und die mit höhenverstellbaren Standfüßen versehen sind. Während bei der Herstellung von Betten die Schwenklager für die Matratzenunterstützung sowie die Befestigung des Schwenkantriebes ohne weiteres möglich ist, bereitet der nachträgliche Einbau in vorhandene Betten erhebliche Probleme. Zum einen sind bis auf die Abmessungen der Matratzen die Abmessungen von Betten nicht genormt, so daß hier unterschiedliche Höhen zu berücksichtigen sind. Des weiteren ist zu berücksichtigen, daß bei den verwendeten Materialien oder auch aufgrund der Bauweise, zum Beispiel bei furnierten oder stoffbespannten Bettkästen, der nachträgliche Einbau der Schwenklager nicht möglich ist, zumal das gesamte Gewicht des Schläfers statt wie bisher von Längsleisten am Bettkasten nur über die beiden stirnseitigen Schwenklager aufgenommen werden muß. Hierzu reichen meist die Abmessungen und auch die Festigkeiten der für den Bau des Bettkastens verwendeten Materialien nicht aus. Die erfindungsgemäße Ausbildung erlaubt es nun, in Form eines Nachrüstsatzes in einen vorhandenen Bettkasten die beiden mit den Schwenklagern versehenen und die Matratzenunterstützung tragenden Stirnteile einzusetzen. Durch entsprechende Einstellung der Höhe der Standfüße kann nun unter Berücksichtigung der Dicke der vorhandenen Matratze die Auflagefläche der Matratzenunterstützung genau auf die Höhe des vorhandenen Bettkastens eingestellt werden. Bei einer mittig gelagerten Matratzenunterstützung ist diese schmaler als die ursprüngliche Matratzenunterstützung, so daß die Matratzenunterstützung frei an den Auflageleisten des Bettkastens sich vorbeibewegen kann. Die Höheneinstellung muß hierbei so vorgenommen werden, daß innerhalb der durch den Schwenkvorgang vorgegebenen Winkelbereich der Schwenkvorgang zusammen mit der aufliegenden Matratze praktisch ungehindert erfolgen kann.

Die mit Standfüßen versehenen Stirnteile können nun mit den entsprechenden Stirnteilen des Bettkastens fest verbunden werden, beispielsweise durch Verschrauben, um eine einwandfreie Abstützung und Ausrichtung der Stirnteile zu gewährleisten. Da in vielen Fällen jedoch die Materialien des vorhandenen Bettkastens nicht geeignet sind, daß hier Schrauben mit größerer Belastung eingeschraubt werden können, ist in einer vorteilhaften' Ausgestaltung der Erfindung vorgesehen, daß die beiden Stirnteile über wenigstens eine Längsstrebe fest miteinander verbunden sind. Hierdurch ergibt sich ein selbsttragender, vom Bettkasten unabhängiger Einsatzteil. Insbesondere bei Matratzenunterstützungen, deren Schwenkachse in der Mittenlängsachse liegt, ist es zweckmäßig, wenn die Längsstrebe unterhalb des Schwenklagers mit dem Stirnteil verbunden ist. Hierdurch ist eine freie Verschwenkbarkeit der Matratzenunterstützung nach beiden Seiten gewährleistet. Um einen derartigen Nachrüstsatz an einen vorhandenen Bettkasten anpassen zu können, ist hierbei ferner vorgesehen, daß die Längsstrebe längenveränderbar ausgebildet ist. Dies kann beispielsweise durch eine geteilte Längsstrebe erreicht werden, die im Teilungsbereich überlappend ausgebildet ist und die in diesem Bereich mit einer Lochreihe und einer Verschraubung durch Schraubbolzen versehen ist. In weiterer Ausgestaltung der Erfindung ist hierbei ferner vorgesehen, daß die Matratzenunterstützung einen Rahmen aufweist, dessen Längsholme und dessen Querholme längenveränderbar ausgebildet sind. Hierdurch ist es möglich, auch die Matratzenunterstützung innerhalb vorgegebener Bereiche an die Abmessungen eines vorhandenen Bettkastens anzupassen.

In zweckmäßiger Ausgestaltung der Erfindung ist hierbei vorgesehen, daß die Längsholme und die Querholme als Rohrprofile ausgebildet sind und daß die Längsholme mit den Querholmen über teleskopartig einschiebbare und lösbare Verbindungsstücke fest miteinander verbunden sind. Der Längenausgleich sowohl der Querholme als auch der Längsholme erfolgt bevorzugt durch den Eckenbereich bildende Winkelstücke, die teleskopartig sowohl in das jeweilige Ende des Querholms und des zugehörigen Längsholms einschiebbar und mit den Holmen befestigbar ausgebildet sind. Auch hier kann die Verbindung wieder über eine entsprechende Lochreihe in den betreffenden Enden der Holme und in den einzuschiebenden Enden der Winkelstücke und durch steckbare Schraubenbolzen erfolgen.

In zweckmäßiger Ausgestaltung der Erfindung ist ferner vorgesehen, daß die Stirnteile in bezug auf die Breite, insbesondere in bezug auf den Breitenabstand der Standfüße veränderbar ausgebildet sind. Diese Anordnung hat den Vorteil, daß die Stirnseiten genau auf die Breite des Bettkastens abgestellt werden können, so daß sich die Stirnseiten bei einem lose eingesetzten Nachrüstsatz seitlich am Bettkasten abstützen und somit die erforderliche Fixierung des Nachrüstsatzes gegen ein seitliches Verschieben bewirken.

In zweckmäßiger Ausgestaltung der Erfindung ist ferner vorgesehen, daß der Rahmen der Matratzenunterstützung mit einem Lattenrost versehen ist, der über Haltemittel mit dem Rahmen verbunden ist. Ein Lattenrost ist nicht nur unter orthopädischen Gesichtspunkten eine ideale Unterstützung für eine Matratze, sondern bietet für ein Bett der erfindungsgemäßen Art auch den Vorteil, daß die durch den Rahmen der Matratzenunterstützung gebildete Ebene unterhalb des Lattenrostes völlig frei ist, so daß hier der erforderliche Freiraum für die Schwenkbewegung nach unten hin vorhanden ist.

In zweckmäßiger Ausgestaltung der Erfindung ist vorgesehen, daß als Schwenklager jeweils ein Pendellager in den Standteilen angeordnet ist, in dem ein mit dem Rahmen verbundener Lagerzapfen gelagert ist. Die Anordnung eines Pendellagers hat insbesondere bei einem Bett der erfindungsgemäßen Art, das durch einen nachträglich eingesetzten Einsatz in einen vorhandenen Bettkasten gebildet wird, den Vorteil, daß durch Montageungenauigkeiten oder auch Bodenunebenheiten bewirkte Fluchtfehler sowie durch Verformungen der Matratzenunterstützung unter Last bewirkte Durchbiegungen ausgeglichen werden, so daß immer eine einwandfreie Schwenkbewegung möglich ist.

Die Erfindung wird anhand schematischer Zeichnungen eines Ausführungsbeispieles näher erläutert. Es zeigen:

Fig. 1 eine Aufsicht auf einen Bettkasten eines normalen Bettes mit eingesetztem Nachrüstsatz,

Fig. 2 einen Schnitt gem. der Linie II-II in Fig. 1 durch ein Stirnteil ohne Darstellung der Matratzenunterstützung,

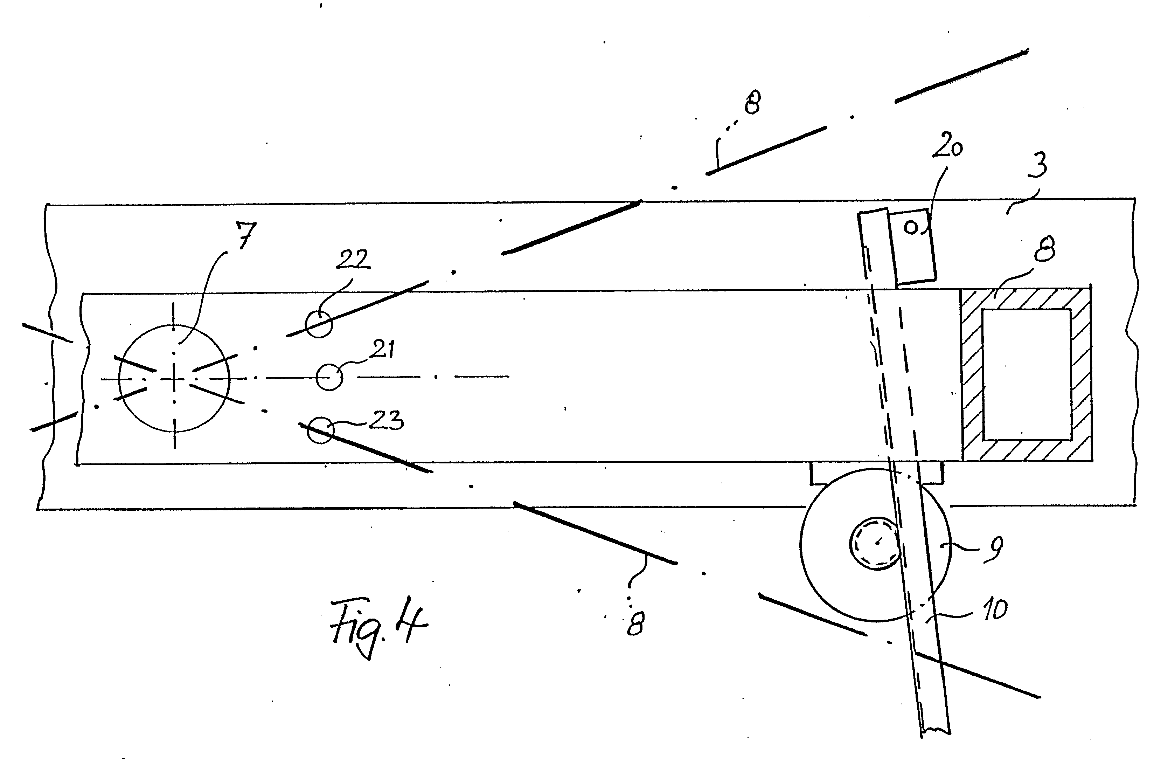

Fig. 3 in größerem Maßstab die Ecke einer Ausführungsform einer in der Länge und in der Breite veränderbaren Matratzenunterstützung,

Fig. 4 eine Stirnansicht auf die Matratzenunterstützung mit Schwenkantrieb,

Fig. 5 eine schematische Darstellung der Schaltungsanordnung,

Fig. 6 in einer Teilansicht in Längsrichtung eine Ausführungsform mit an der Matratze angreifendeny Halteelementen,

Fig. 7 schematisch eine Schwenklagerung der Matratzenunterstützung über höhenverstellbare Füße.

Als Ausführungsbeispiel wird ein sogenannter Nachrüstsatz beschrieben, der es ermöglicht, ein vorhandenes Bett in ein Bett für Schnarcher umzurüsten, was insbesondere bei wertvollen Möbeln wünschbar ist. Der Teil eines Bettes wird nachfolgend unabhängig von der jeweiligen Bauform als Bettkasten 1 bezeichnet. Im Hinblick auf eine übersichtlichere Darstellung ist der Bettkasten 1 gestrichelt dargestellt.

Die Umrüstung erfolgt in der Weise, daß die vorhandene Matratzenunterstützung, beispielsweise ein Sprungfederrahmen, aus dem Bett herausgenommen wird und dann der Nachrüstsatz in den Bettkasten eingesetzt wird. Der Nachrüstsatz ist hierbei so konzipiert, daß er eine in sich geschlossene, selbsttragende Baueinheit darstellt. Außer der Montage und die nachstehend noch näher beschriebene Anpassung an die Abmessungen des Bettkastens 1 sind keine zusätzlichen Montagemaßnahmen erforderlich, wie beispielsweise die Verbindung von Teilen des Nachrüstsatzes mit dem Bettkasten 1.

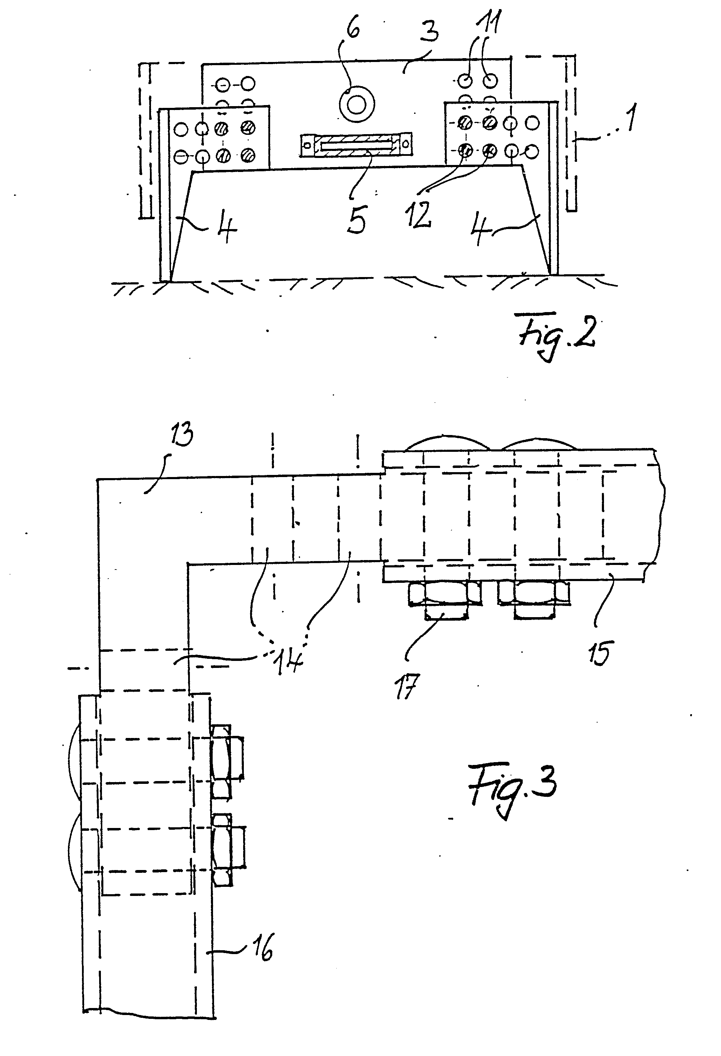

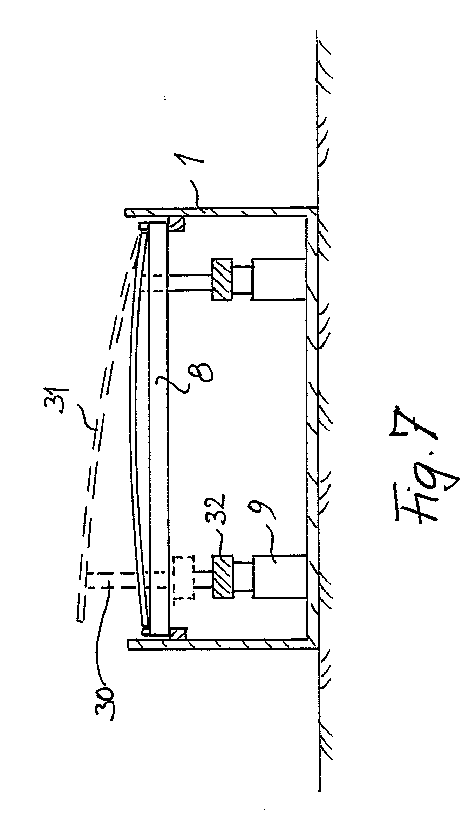

Der Nachrüstsatz wird im wesentlichen gebildet durch zwei Stirnteile 2 und 3, die jeweils mit Füßen 4 (Fig. 2) versehen sind. Die beiden Stirnteile 2 und 3 sind durch eine Längsstrebe 5 fest miteinander verbunden, wobei die Längsstrebe 5 unterhalb der Längsmittelachse verläuft. In den beiden Stirnteilen 2 und 3 sind jeweils Schwenklager 6 eingesetzt, die als Pendellager ausgebildet sind und die einen Lagerzapfen 7 einer als Rahmen ausgebildeten Matratzenunterstützung 8 aufnehmen. Die Lagerzapfen 7 liegen wiederum in der Längsmittelachse der Matratzenunterstützung 8, so daß diese nach beiden Seiten hin verschwenkbar ist. Statt der Lagerung über die Lagerzapfen 7 kann die Matratzenunterstützung 8 an ihren Stirnenden jeweils über zwei Gelenkhebel abgestützt sein, deren anderes Ende jeweils in Bodennähe am Stirnteil 2 bzw. 3 angelenkt ist. Die Gelenkhebel an jeder Stirnseite sind unter einem Winkel zueinander ausgerichtet und bilden jeweils eine Vier-Gelenk-Kette, wie sie in Fig. 7 schematisch angedeutet ist. Die Neigung der Gelenkhebel 32 gegenüber der Horizontalen und der Abstand der Gelenkpunkte 33, 34 bzw. 35, 36 zueinander bestimmt das Verhältnis von Schwenkweg der Hebel und Neigung der Matratzenunterstützung.

Die Matratzenunterstützung 8 ist über einen Schwenkantrieb 9, beispielsweise einen elektrischen Getriebemotor, der über eine beispielsweise als Zahnstange ausgebildete Schubstange 10, die am Stirnteil 3 angelenkt ist, aus der horizontalen Lage nach beiden Seiten hin in eine Schräglage verschwenkbar.

Da die lichten Abmessungen vorhandener Bettkästen abgesehen von den unterschiedlichen Abstufungen der Matratzengröße je nach Konstruktion der Auflage der Matratzenunterstützung uneinheitlich sind, ist der Nachrüstsatz so konzipiert, daß er insgesamt sowohl in der Breite als auch in der Länge sowie hinsichtlich der Höhe einstellbar ist. Die Anordnung kann auch so getroffen werden, daß der Getriebemotor 9 am Stirnteil bzw. am Bettkasten angeordnet ist und die Schubstange 10 an der Matratzenunterstützung angelenkt ist. Anstelle einer Zahnstange kann die Schubstange auch als Gewindespindel ausgebildet sein, der eine entsprechende Spindelmutter zugeordnet ist. Je nach Ausbildung des Antriebs kann die Spindel oder die Spindelmutter drehbar und über den Motor antreibbar sein. Bei einer Lagerung der Matratzenunterstützung über Gelenkhebel kann die Schubstange auch auf einem der Gelenkhebel einwirken (Vgl. Pfeil 37 in Fig. 7).

Wie Fig. 2 zeigt, sind die Stirnteile 2 und 3 so breit bemessen, daß sie in den schmalsten der im Markt befindlichen Bettkästen einsetzbar sind. Hierzu sind jeweils im Endbereich eine Vielzahl von Durchgangslöchern 11 in den Stirnteilen angeordnet, die mit Durchgangslöchern 12 in den Standfüßen 4 korrespondieren. Auf diese Weise ist es möglich, die Höhe der durch die Schwenklager 6 definierten Längsmittelachse der Anordnung gegenüber dem Fußboden bzw. in bezug auf den vorhandenen Bettkasten 1 durch entsprechenden Höhenversatz der Füße 4 gegenüber den Stirnteilen 2 und 3 einzustellen. Die Standfüße 4 können hierbei gleichzeitig auch in der Breite so eingestellt werden, daß diese mit ihren Außenseiten an den Innenseiten des Bettkastens 1 anliegen, so daß das eingesetzte Teil im Bettkasten 1 gegen seitliche Verschiebungen fixiert ist.

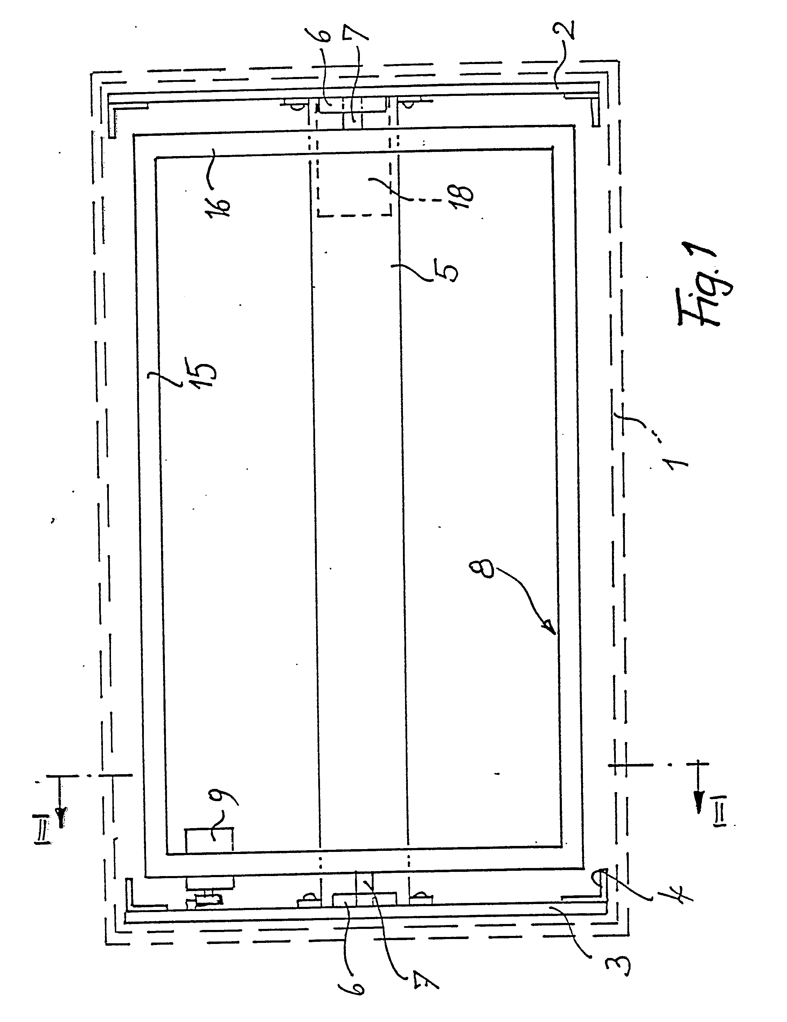

Die bei dem dargestellten Ausführungsbeispiel als einfacher Rahmen ausgebildete Matratzenunterstützung ist vorzugsweise aus einem Rohrprofil hergestellt. Um auch hier die erforderliche Anpassung in der Breite und in der Länge zu erzielen, sind jeweils im Eckenbereich als Verbindungsstücke 13 Winkelstücke vorgesehen, die in vorgegebenen Abständen mit Querbohrungen 14 versehen sind. Diese Winkelstücke sind stirnseitig jeweils in den Rahmen bildende Längsholme 15 und Querholme 16 eingeschoben, die mit wenigstens zwei Querbohrungen versehen sind. Das Verbindungsstück 13 wird nun entsprechend der geforderten Längenabmessung teleskopartig in den Längsholm 15 und. oder in den Querholm 16 so weit eingeschoben, bis die erforderliche Abmessung gegeben ist. Die Winkelstücke 13 werden dann mit den Querholmen 16 und den Längsholmen 15 fest verbunden, beispielsweise durch Schraubbolzen 17. Die Längsstrebe 5 ist in gleicher Weise mit einem teleskopartig gegenüber dem Strebenteil verschiebbaren Verbindungsstück 18 versehen, wie dies in Fig. 1 schematisch angedeutet ist.

In Fig. 4 ist schematisch die Anordnung und die Funktionsweise des Schwenkantriebs näher erläutert. Bei dem darge stellten Ausführungsbeispiel wird der Schwenkantrieb durch einen elektrischen Getriebemotor 9 gebildet, dessen Antriebswelle ein Ritzel 19 trägt, das mit einer Zahnstange 10 zusammenwirkt. Der Getriebemotor 9 ist hierbei zweckmäßigerweise auf der Unterseite der Matratzenunterstützung 8 angeordnet, um eine möglichst große Raumausnutzung des vorhandenen Bettkastens zu erzielen. Die als Zahnstange ausgebildete Schubstange 10 ist entsprechend am zugeordneten Stirnteil 2 bzw. 3 angelenkt und wird in üblicher Weise durch einen entsprechenden Käfig am Getriebemotor 9 mit dem Ritzel 19 in Eingriff gehalten. Fig. 4 zeigt die Anordnung in der Horizontallage. Je nach Drehrichtung des Getriebemotors 9 kann nun die Matratzenunterstützung 8 um einen Winkel zwischen 10° und 25° gegenüber der Horizontalen jeweils nach oben oder nach unte verschwenkt werden.

Die Anlenkung der Schubstange 10 am Stirnteil 2 oder 3 ist mit einem lastfühlenden Umsteuerschalter 20 versehen, der so ausgebildet ist, daß bei einer Belastung der Schubstange 10 durch ein Auflastmoment im Uhrzeigersinne die Drehrichtung des Getriebemotors 9 so geschaltet ist, daß beim Einschalten die Matratzenunterstützung gegen den Uhrzeigersinn hochgeschwenkt wird. Bei einer Belastung der Schubstange 10 durch ein gegen den Uhrzeigersinn wirkendes Lastmoment wird die Drehrichtung über den Umsteuerschalter 20 so angesteuert, daß ein Verschwenken der Matratzenunterstützung im Uhrzeigersinne erfolgt.

Die elektrische Steuerschaltung weist ferner noch Endlagenschalter auf, die den Getriebemotor bei einer Betätigung jeweils abschaltet, wenn die jeweilige Schräglage erreicht ist, aber auch wenn die Matratzenunterstützung aus der Schräglage wieder in die Horizontallage zurückgeschwenkt wird. Hierzu sind drei Endlagenschalter vorgesehen, die zweckmäßigerweise als berührungslos arbeitende Endlagenschalter ausgebildet sind. Dies hat gegenüber üblichen Endlagenschaltern den Vorteil, daß keine Wartung erforderlich ist und so Störungen durch den in Schlafräumen nicht vermeidbaren Staubanfall ausgeschlossen sind. Es ist aber auch möglich, entsprechend gekapselte Endlagenschalter einzusetzen. In Fig. 4 sind drei Endlagenschalter 21, 22 und 23 angedeutet. Hierzu ist jeweils ein Teil des Schaltelementes an einem Stirnteil und das zugeordnete Schaltelement an der Matratzenunterstützung angeordnet. Da es sich hier um Endlagenschalter üblicher Bauart handelt, sind diese nur schematisch angedeutet.

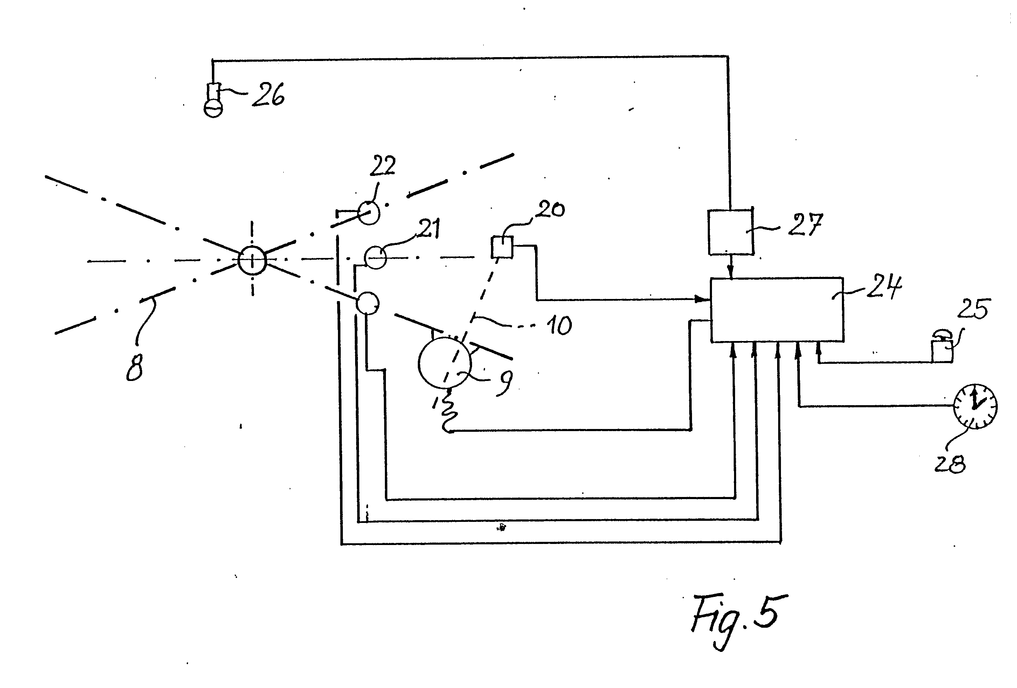

In Fig. 5 ist schematisch die Zuordnung der einzelnen Schaltelemente im Rahmen einer Steuerschaltung dargestellt. Auf eine entsprechend ausgebildete elektrische Steuerschaltung 24 sind zum einen die drei Endlagenschalter 21, 22 und 23 so aufgeschaltet, daß sie jeweils beim Erreichen der durch die Endlagenschalter definierten Position den Motor abschalten. Zugleich wird in üblicher Weise jedoch ein zugehörigen Schalter in der Steuerschaltung betätigt, der ein Wiederanlaufen des Motors in Gegenrichtung erlaubt.

Der lastfühlende Schalter 20 an der Anlenkung der Schubstange 10 ist ebenfalls auf die Steuerschaltung 24 aufgeschaltet und so geschaltet, daß je nach dem durch die Belastung der Matratzenunterstützung vorgegebenen Lastmoment, das sich in Abhängigkeit von der jeweiligen Lage des Schläfers ändern kann, eine Drehung des Motors 9 gegen die Richtung des Lastmomentes erfolgt, sobald der Motor eingeschaltet wird.

Der Motor kann hierbei durch einen Schalter 25, vorzugsweise einen Tastschalter, in Betrieb gesetzt werden. Bei dem dargestellten Ausführungsbeispiel ist ferner noch ein elektro-akustischer Sensor 26 vorgesehen, beispielsweise in Form eines kleinen Mikrofones, der auf eine Meßschaltung 27 aufgeschaltet ist, deren Signalausgang auf die Steuerschaltung 24 aufgeschaltet ist. Die Meßschaltung 27 kann hierbei so ausgebildet sein, daß sie ein Schaltsignal abgibt, wenn über den elektroakustischen Sensor ein Geräusch erfaßt wird, das eine vorgegebene Lautstärke überschreitet. Zweckmäßig ist es jedoch, die Meßschaltung so auszulegen, daß ein Steuersignal nur dann ausgelöst wird, wenn Geräusche mit einer bestimmten Frequenz und/oder einer vorgegebenen Lautstärke über den elektro-akustischen Sensor 26 erfaßt werden. Da Schnarchgeräusche eine sehr niedrige Frequenz aufweisen, besteht somit die Möglichkeit, dieses Frequenzband auszufiltern, so daß alle übrigen Geräusche nicht zur Auslösung eines Steuersignals führen.

Über eine mechanische oder elektrische Kurzzeitschaltuhr 28, die ebenfalls über eine entsprechende Schaltungsanordnung mit der Steuerschaltung 24 verbunden ist, wird erreicht, daß nach Ablauf eines vorgegebenen Zeitraumes von beispielsweise 5 bis 10 Minuten, die Matratzenunterstützung aus der Schräglage wirder in die Horizontallager zurückgeschwenkt wird, so daß der nunmehr in der Seitenlage befindliche Schläfer ungestört weiterschlafen kann. Dies ist deshalb von Bedeutung, weil bei einem Halten der Matratzenunterstützung in der Schräglage über einen längeren Zeitraum der Schlaf aufgrund der ungewohnten Lage und der Tendenz des entspannt auf der Matratze liegenden Körpers "abwärts" zu rollen, der Schlaf unterbrochen wird, zumindest jedoch innerhalb kurzer Zeit aufgrund der Muskelreaktionen die Schlaftiefe reduziert wird.

Zur Zuordnung der Endlagenschalter zur Matratzenunterstützung können diese auch in den Antriebsmechanismus integriert werden. Die jeweilige Schräglage läßt sich auch von der Stellung der Schub- bzw. Zahnstange zum Antriebsmotor abgreifen.

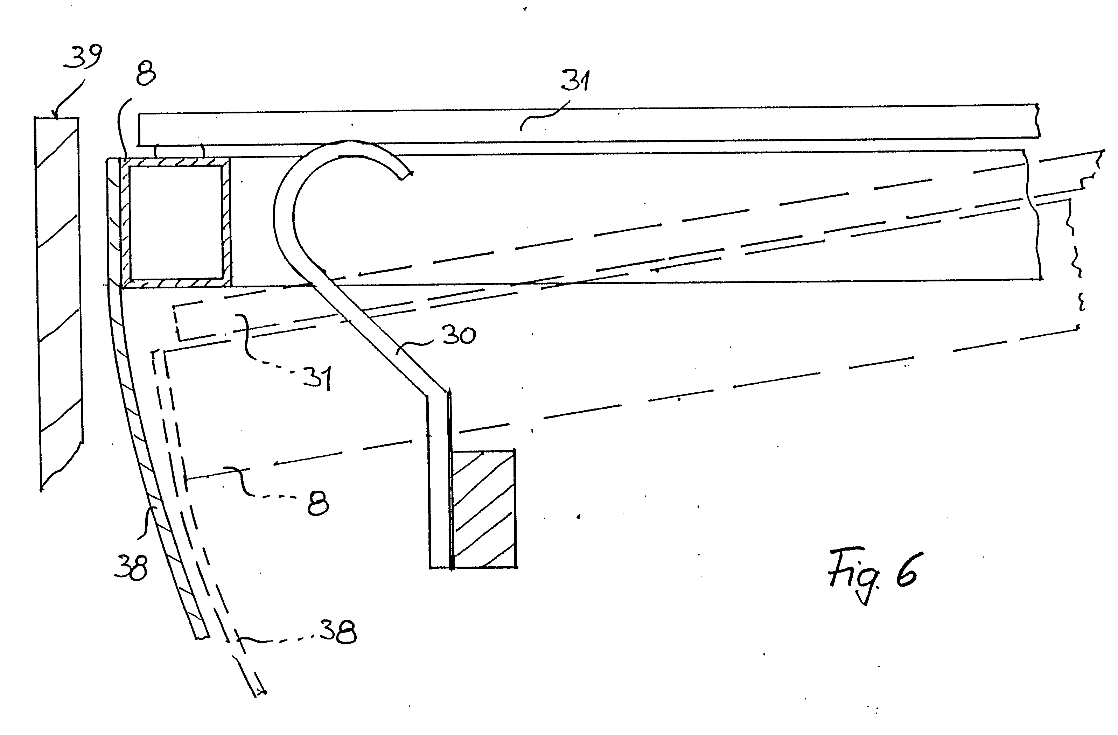

Wie die Teilansicht gem. Fig. 6 zeigt, können entsprechend einer Ausgestaltung feststehende kammartig angeordnete Halteelemente 30 den beiden Längsrändern der als Lattenrost ausgebildeten Matratzenunterstützung zugeordnet sein. Die einzelnen Halteelemente 30 ragen von unten in den Zwischen raum zwischen je zwei Latten 31 hinein und enden bei horizontaler Stellung der Matratzenunterstützung mit ihren oberen Enden vorzugsweise etwas unterhalb der Auflagefläche des Lattenrostes.

Wird die Matratzenunterstützung 8 verschwenkt, was in Fig. 6 gestrichelt dargestellt ist, dann wird der Rand der aufliegenden Matratze von den Halteelementen 30 in der ursprünglichen Höhe gehalten, so daß die Matratze im Randbereich leicht rinnenförmig verformt wird. Relativ zur Liegefläche der Matratze ist dann der Randbereich leicht nach oben aufgebogen, so daß sich eine einwandfreie Abstützung des Körpers in der Seitenlage ergibt. Hierdurch wird für den Schläfer in der Schwenkstellung das Gefühl vermieden, er könne aus dem Bett fallen.

Aus der Darstellung gem. Fig. 6 ist zu erkennen, daß die Bewegungsabläufe auch umgelenkt werden können. Insbesondere bei einem Lattenrost bieten die Halteelemente 30 die Möglichkeit, den Lattenrost wie üblich in den Bettkasten einzulegen und die Halteelemente mit dem Antrieb zu verbinden, so daß beim Ansprechen der Steuerung die Halteelemente die Matratze randseitig hochheben. Bei weichen Matratzen muß hier eine entsprechend starre Unterstützung vorgesehen werden, um einen genügend breiten Bereich der Matratze in die Schräglage zu bringen, der die Veränderung der Körperlage bewirkt. Diese Unterstützung ist zweckmäßig an oder nahe an der anderen Längsseite angelenkt. Diese Unterstützung kann auch durch Latten des Lattenrostes gebildet werden. Hierbei sind die einzelnen Latten wechselnd an dem einen und an dem anderen Längsholm des Rahmens gelenkig gehalten. Das andere Ende ist dann jeweils auf einem heb- und senkbaren Halteelement im Bereich der anderen Längsseite abgestützt. In diesem Fall greifen die Halteelemente nicht durch die Zwischenräume hindurch. Den Halteelementen 30 auf jeder Längsseite ist jeweils ein Antrieb zugeordnet, der beispielsweise durch einen Schubstangenantrieb der vorerwähnten Bauform gebildet werden kann. Durch entsprechende lastfüh lende Sensoren in der Steuerschaltung muß auch hier wieder dafür gesorgt werden, daß die "richtige" Seite hochschwenkt, also die Seite, auf der das größere Gewicht liegt.

Bei anders gestalteten Matratzenunterstützungen müssen entsprechende Ausnehmungen vorgesehen werden, die den Durchtritt der Halteelemente ermöglichen. Die Anordnung der Halteelemente muß so ausgebildet sein, daß die freie Schwenkbarkeit der Matratzenunterstützung nicht beeinträchtigt, die Matratze jedoch am Rand sicher gehalten wird.

Um Verletzungen zu vermeiden, ist an den jeweils nach oben verschwenkbaren Teilen der Matratzenunterstützung 8 eine nach unten weisende, umlaufende Abdeckschürze 38 angeordnet. Wenn der Rand der Matratzenunterstützung über die Kante 39 des Bettkastens 1 hochgeschwenkt wird, dann wird die Bildung eines Spaltes vermieden, durch den unbeabsichtigt hindurchgegriffen werden könnte.

Bei einer Schwenklagerung der Matratzenunterstützung über Lagerzapfen, wie in Fig. 1 dargestellt, kann die Abdeckschürze 38 aus einem starren Material, beispielsweise aus Kunststoff, bestehen.

Bei einer Schwenklagerung über Gelenkhebel können nur an den stirnseitigen Rändern starre Formteile angeordnet werden, da der Schwenkvorgang auch eine Horizontalverschiebungskomponente enthält. An den Längsrändern sind dann Abdeckschürzen aus einem biegsamen Material vorzusehen, beispielsweise eine rouleauartige Textilschürze, die ausreichend straff gespannt bleibt.

Wie die Darstellung in Fig. 7 zeigt, kann der Antrieb 9, spielsweise in Form vertikal ausgerichteter Schubstangen, jeweils durchgehend an Traversen 32 angreifen, mit denen die Halteelemente 30 verbunden sind. Auf diesen stützen sich dann abwechselnd die Latten 31 des Lattenrostes ab, so daß jeweils die eine Hälfte der Latten nach rechts, wie dargestellt, und die andere Hälfte nach links hochgeschwenkt werden kann, je nach Ansteuerung durch die Steuereinrichtung. Der Rahmen 8 liegt im Bettkasten 1 fest auf.

Anstelle der einzelnen Latten kann der Rahmen 8 insgesamt entweder rechts oder links hochgeschwenkt werden. Hierbei ist es auch möglich, die Antriebe mit ihren Schubstangen zugleich als Füße für den Rahmen vorzusehen. Die Traverse 32 kann aber auch durch einen Schwenkantrieb auf- und abbewegt werden.

## Ansprüche

1. Bett zum Beeinflussen des Schnarchens, das eine um wenigstens eine Längsachse verschwenkbar ausgebildete Matratzenunterstützung (8) mit im wesentlichen ebenflächiger Liegefläche aufweist, die mit einem vorzugsweise elektromotorischen Schwenkantrieb (9) verbunden ist, der über eine Steuerschaltung (24) an eine Stromversorgung angeschlossen ist,
dadurch **gekennzeichnet,**
daß der Matratzenunterstützung (8) vorzugsweise berührungslos arbeitende Endlagenschalter (21, 22, 23) zugeordnet sind, die jeweils bei Erreichen der Schräglage oder der Horizontallage den Antrieb (9) abschalten.

2. Bett nach Anspruch 1, mit einer um wenigstens eine Längsachse, vorzugsweise die Mittellängsachse aus der Horizontallage verschwenkbar gelagerten Matratzenunterstützung, dadurch gekennzeichnet, daß ein lastfühlender Umschalter (20) für den Antrieb (9) vorgesehen ist, der jeweils die Richtung einer von der Horizontallage ausgehenden Schwenkbewegung gegen das von einer einseitigen Auflast auf der Matratzenunterstützung (8) vorgegebene Lastmoment steuert.

3. Bett nach Anspruch 1 oder 2, dadurch gekennzeichnet, daß als Schwenkantrieb ein elektrischer Getriebemotor (9) vorgesehen ist, der auf eine Schubstange (10) einwirkt, die mit der Matratzenunterstützung in Verbindung steht.

4. Bett nach Anspruch 1, 2 oder 3, dadurch gekennzeichnet, daß der Getriebemotor (9) stirnseitig unter der Matratzenunterstützung (8) mit Abstand zur Schwenkachse angeordnet ist.

5. Bett nach Anspruch 4, dadurch gekennzeichnet, daß insbesondere bei einer um die Mittellängsachse schwenkbar gelagerten Matratzenunterstützung (8) die Schubstange (10) mit dem in Richtung des Lastmomentes vorgebenden lastfühlenden Umschalter (20) der Steuerschaltung (24) verbunden ist.

6. Bett nach einem der Ansprüche 1 bis 5, dadurch gekennzeichnet, daß eine Kurzzeitschaltuhr (28) vorgesehen ist, die mit der Steuerschaltung (24) des Schwenkantriebs verbunden ist, die beim Verschwenken der Matratzenunterstützung (8) aus der Horizontallage in die Schräglage geschaltet wird und die nach Ablauf einer vorgegebenen Zeit den Schwenkantrieb auf Rücklauf in die Horizontallage schaltet.

7. Bett nach einem der Ansprüche 1 bis 6, dadurch gekennzeichnet, daß ein elektro-akustischer Sensor (26) auf die Steuerschaltung (24) aufgeschaltet ist, der bei Schnarchlauten den Schwenkantrieb einschaltet.

8. Bett nach einem der Ansprüche 1 bis 7, dadurch gekennzeichnet, daß zu beiden Längsseiten der Matratzenunterstützung (8) feststehende Halteelemente (30) für den Rand der Matratze angeordnet sind.

9. Bett nach einem der Ansprüche 1 bis 8, dadurch gekennzeichnet, daß die Matratenunterstützung in an sich bekannter Weise als Lattenrost mit quer zur Längsachse im Abstand zueinander angeordneten Latten ausgebildet ist und daß am Bett den Längsrändern der Matratzenunterstützung (8) zugeordnete, Halteelemente (30) angeordnet sind, die jeweils in den Zwischenraum zwischen zwei Latten (31) von unten hineinragen und bei Horizontalstellung der Matratzenunterstützung (8) mit ihren oberen Enden im wesentlichen in der Ebene des Lattenrostes liegen.

10. Bett für Schnarcher in Form eines Nachrüstsatzes für vorhandene Betten insbesondere Bett nach einem der Ansprüche 1 bis 9, dadurch gekennzeichnet, daß zwei Stirnteile (2, 3) vorgesehen sind, die jeweils ein Schwenklager (6) für die Matratzenunterstützung (8) aufweisen und die mit höhenverstellbaren Standfüßen (4) versehen sind.

11. Bett nach Anspruch 10, dadurch gekennzeichnet, daß die beiden Stirnteile (2, 3) über wenigstens eine Längsstrebe (5) miteinander verbunden sind.

12. Bett nach Anspruch 10 oder 11, dadurch gekennzeichnet, daß die Längsstreben (5) jeweils unterhalb des Schwenklagers (6) mit dem Stirnteil (2, 3) verbunden sind.

13. Bett nach Anspruch 11 oder 12, dadurch gekennzeichnet, daß die Längsstrebe (5) in ihrer Länge veränderbar ausgebildet ist.

14. Bett nach einem der Ansprüche 10 bis 13, dadurch gekennzeichnet, daß die Matratzenunterstützung (8) einen Rahmen aufweist, dessen Längsholme (15) und dessen Querholme (16) längenveränderbar ausgebildet sind.

15. Bett nach einem der Ansprüche 10 bis 14, dadurch gekennzeichnet, daß die Längsholme (15) und die Querholme (16) als Rohrprofile ausgebildet sind und daß die Längsholme (15) mit den Querholmen (16) über teleskopartig einschiebbare, lösbare Verbindungsstücke (13) fest miteinander verbunden sind.

16. Bett nach Anspruch 15, dadurch gekennzeichnet, daß zumindest für den Längenausgleich der Querholme (16) jeweils ein teleskopartig in den Querholmen (16) einschiebbares, den Eckenbereich bildendes Winkelstück (13) vorgesehen ist.

17. Bett nach einem der Ansprüche 10 bis 16, dadurch gekennzeichnet, daß die Stirnteile (2, 3) in bezug auf die Breite, insbesondere in bezug auf den Breitenabstand der Standfüße (4), veränderbar ausgebildet sind.

18. Bett nach einem der Ansprüche 10 bis 17, dadurch gekennzeichnet, daß der die Matratzenunterstützung (8) bildende Rahmen mit einem Lattenrost versehen ist, der über Haltemittel mit dem Rahmen verbunden ist.

19. Bett nach einem der Ansprüche 10 bis 18, dadurch gekennzeichnet, daß als Schwenklager (6) jeweils ein Pendellager in den Stirnteilen (2, 3) angeordnet ist, in dem ein mit der Matratzenunterstützung (8) verbindbarer Lagerzapfen (7) gelagert ist.

20. Bett nach einem der Ansprüche 10 bis 19, dadurch gekennzeichnet, daß die durch den Lagerzapfen (7) definierte Schwenkachse in der Längsmittelachse der Matratzenunterstützung (8) liegt.

21. Bett nach einem der Ansprüche 10 bis 19, dadurch gekennzeichnet, daß die durch den Lagerzapfen (7) definierte Schwenkachse der Matratzenunterstützung (8) seitlich und mit Abstand parallel zur Längsmittelachse der Matratzenunterstützung (8) liegt.

22. Bett nach einem der Ansprüche 1 bis 21, dadurch gekennzeichnet, daß die Matratzenunterstützung (8) am Rand der beim Schwenken die Horizontalebene überragenden Bereiche mit einer nach unten in den Bettkasten (1) reichenden Abdeckschürze (38) versehen ist.

Fig. 1

EP 0 355 397 A1

Fig.2

Fig.3

Fig. 4

Fig.5

EP 0 355 397 A1

Fig 6

Fig.7

EP 0 355 397 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | DE-U-8 200 020 (I. WITTMER) * Anspruch 1; Seite 6, Zeile 20 - Seite 7, Zeile 10; Figuren 1,2 * --- | 1,2 | A 61 F 5/56 A 61 G 7/00 A 47 C 19/00 |
| A | PATENT ABSTRACTS OF JAPAN Band 9, Nr. 113 (P-356)(1836), 17. Mai 1985; & JP - A - 60 410 (FRANCE BED K.K.) 05.01.1985 --- | 1,7 | |
| A | GB-A-1 554 107 (H. STEEPER LTD.) * Ansprüche 1,2; Figuren 1-3 * --- | 1,2 | |
| D,A | DE-U-1 837 239 (G. LOHS) * Ansprüche 1-3; Seite 3, Zeilen 1-12 * --- | 1,2 | |
| A | CH-A- 269 940 (G. BISCHOFF) * Anspruch; Figuren 2,3 * --- | 1,3,4 | |
| P,A | EP-A-0 294 510 (L. FUCHS) * Anspruch 1 * --- | 1 | RECHERCHIERTE SACHGEBIETE (Int. Cl.5) |
| D,A | US-A-3 089 130 (G. J. WILSON) * Figuren 1,2 * ----- | 1,7 | A 47 C A 61 F A 61 G |

Der vorliegende Recherchenbericht wurde für alle Patentansprüche erstellt

| Recherchenort | Abschlußdatum der Recherche | Prüfer |
|---|---|---|
| BERLIN | 27-09-1989 | KANAL P K |